(19) Europäisches Patentamt

European Patent Office

Office européen des brevets

(11) **EP 2 489 302 B1**

(12) **EUROPEAN PATENT SPECIFICATION**

(45) Date of publication and mention
of the grant of the patent:
**24.03.2021 Bulletin 2021/12**

(51) Int Cl.:
**A61B 5/0205** (2006.01) **A61B 5/22** (2006.01)

(21) Application number: **12155792.0**

(22) Date of filing: **16.02.2012**

(54) **Method and device for estimating energy consumption**

Verfahren und Vorrichtung zur Schätzung des Energieverbrauchs

Procédé et dispositif permettant d'estimer la consommation d'énergie

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.02.2011 FI 20115150
17.02.2011 US 201161443731 P**

(43) Date of publication of application:
**22.08.2012 Bulletin 2012/34**

(73) Proprietor: **Suunto Oy
01510 Vantaa (FI)**

(72) Inventors:
• **Martikka, Mikko
FI-01510 Vantaa (FI)**
• **Lindman, Erik
FI-01510 Vantaa (FI)**

(74) Representative: **Laine IP Oy
Porkkalankatu 24
00180 Helsinki (FI)**

(56) References cited:
**EP-A2- 0 072 235        WO-A2-2009/131664
US-A- 5 810 722         US-A1- 2007 265 534
US-A1- 2009 018 405     US-B1- 7 431 696**

• **REID ET AL: "Poor agreement between
continuous measurements of energy
expenditure and routinely used prediction
equations in intensive care unit patients",
CLINICAL NUTRITION, CHURCHILL
LIVINGSTONE, LONDON, GB, vol. 26, no. 5, 1
October 2007 (2007-10-01), pages 649-657,
XP022312391, ISSN: 0261-5614, DOI:
10.1016/J.CLNU.2007.02.003**

EP 2 489 302 B1

**Description**

**Field of the invention**

The present invention relates to a method and apparatus for estimating the energy consumption of a human body. Especially the invention relates to defining the energy consumption of an exercise carried out at a low intensity level. In the method, heart rate is measured by means of a sensor for providing rate data, the respiratory frequency of the person is determined on the basis of the rate data and further the energy consumption of the person is determined on the basis of the respiratory frequency,

**Prior Art**

Accurately determining the energy consumption during an exercise requires determining or estimating the frequency and depth of respiration. Ventilation can be calculated as product of these, and ventilation can further be used for determining the level of metabolism of a person and thus for estimating the consumption of energy. Earlier patent literature and other literature disclose some different methods for calculating both the individual intermediate stages and the final energy consumption. The following discussion relates to two publications representing probably the closest prior art.

US patent 5810722 discloses a method by means of which it is possible to determine metabolic thresholds of a person as well as principles by means of which the ventilation can be estimated. The preamble of the patent discloses that the depth of respiration is a nearly linear function of physical intensity and that ventilation is the product of respiratory frequency and depth of respiration. The said publication does not discuss estimation of the depth of respiration in detail. According to this publication, the respiratory frequency can be determined on the basis of heart rate variation. The publication especially discusses a method in which a person is instructed to exercise with an increasing intensity and in which the threshold between aerobic and anaerobic exercise is determined by a) measuring the heart rate during the test, b) measuring inter-beat intervals during the test, c) determining respiratory frequency from inter-beat interval variation and d) determining at least one metabolic threshold on the basis of heart rate and respiratory frequency. Additionally, in the method it is possible to e) estimate the depth of respiration from the magnitude of inter-beat interval variation, f) determine ventilation as a function of heart rate, the ventilation being derived from respiratory frequency and estimated depth of respiration and g) determine at least one metabolic threshold on the basis of ventilation and heart rate. In the publication the inter-beat interval values are produced by means of the R spikes of the heart rate signal, with a timing accuracy in the range of 1 ms.

US publication 2005/0209521 refers to the above-mentioned patent and it states that the disclosed method is best suited for an analysis of a static situation and that no accurate analysis methods are disclosed there. US publication 2005/0209521 discloses another, relatively complex way of calculating respiratory frequency and depth of respiration. As is disclosed in the publication, there are many known methods by means of which time series can be converted into frequency form (such as Fourier transform) and by means of which respiratory frequency can be estimated. Additionally it is stated that ventilation is provided as product of respiratory frequency and depth of respiration, but that previously no method has been disclosed for providing depth of respiration from heart rate data only. According to the publication this is because the person's weight, height etc. have an effect on the vital capacity, whereby the method disclosed in US patent 5810722 is only an estimate of the correct depth of respiration. It is further stated in the US publication 2005/0209521 that there are many ways to use the methods for estimating the depth of respiration disclosed in said publication, but essentially these methods utilize heart rate information and parameters describing a person. The starting data of the flow chart shown in US publication 2005/0209521 are heart rate, depth of respiration and background parameters. The publication also teaches that ventilation can be calculated directly from heart rate data and respiratory frequency using a number of mathematical ways (e.g. neural computation).

In brief, in the method according to the US publication 2005/0209521, a unit RFD1 describing respiratory frequency is calculated from heart rate data using inter-beat interval variation and at least a second component RFD2 determining respiratory frequency is calculated from heart rate information. All components thus calculated are combined with an expert function i.e. with a neural network according to the invention, into respiratory frequency. RFD1 describes an optimal steady state situation, while RFD2 discloses a temporal variation of respiratory frequency. According to the publication, the depth of respiration can be determined from a heart rate sequence. Ventilation is determined by a) multiplying depth of respiration by respiratory frequency, b) calculating at least one additional parameter from heart rate data and c) combining the values provided thus into ventilation using a mathematical function. Other relevant prior art is disclosed in EP0072235 A2, WO 2009/131664 A2, US 7431696 B1, REID ET AL: "Poor agreement between continuous measurements of energy expenditure and routinely used prediction equations in intensive care unit patients", CLINICAL NUTRITION, CHURCHILL LIVINGSTONE, LONDON, GB, vol. 26, no. 5, 1 October 2007, pages 649-657, US 2009/0018405 A1, US 2007/0265534 A1.

The above-described methods are usable as such, but they also include considerable disadvantages. A considerable disadvantage for the user is that the energy consumption estimates provided by these at especially low exercise intensities are relatively unsure and inaccurate. It has especially been noticed that low intensity energy consumption estimates for persons with a high body weight index, especially overweight persons, are inaccurate and can considerably deviate from actual energy consumption. Using the normally used methods the error can be as high as 500 to 1000 kcal/d.
Thus there is a need for improved energy consumption estimation methods.

**Summary of the invention**

An aim of the invention is to provide a more accurate method of determining energy consumption, especially for low intensity, i.e. mainly for the range of working and useful exercise corresponding with normal everyday life.
The invention is based on the idea that when certain body weight (index) criteria are met, the energy consumption is not calculated directly from the actual mass of the person, but instead the energy consumption is corrected downwards using a formula taking into account the deviation of the mass from the predetermined value.
In the method, a first threshold is chosen for the mass of a person and if the mass of a person is larger than the first threshold value, energy consumption is calculated with a formula, taking into account the deviation of the mass of the person from the predetermined value, preferably especially from the said first threshold value.
More specifically, the invention is characterized by what is stated in the independent claims.
A second threshold value is selected for the intensity of the exercise and in case the intensity determined by the heart rate data of the exercise is lower than the second threshold value and the mass of the person is higher than the first threshold value, energy consumption is calculated with a formula taking into account the deviation of the mass of the person and the intensity of the exercise from the first and second threshold value, correspondingly.
The correction is effected by using a so-called effective mass, smaller than the actual mass. More specifically, in the method

- a first threshold is selected for the mass of a person,

- a second threshold is selected for the intensity of the exercise,

- in case the mass of the person is larger than the first threshold value and the intensity of the exercise optionally determined using the heart rate data of the exercise is lower than the second threshold value, the energy consumption is calculated using a formula in which the factor is the effective mass of a person, the effective mass being smaller than the actual mass.

The effective mass approaches the actual mass as the intensity of the exercise approaches the second threshold value.
The intensity of the exercise, and accordingly the second threshold value, can be determined on the basis of heart rate frequency, respiratory frequency or ventilation. Preferably the second threshold value is selected from the range 1.5 MET to 3.0 MET, which is in the aerobic range of the person, preferably about 2 MET (metabolic equivalent of task, 1 MET = 3.5 mlO2/kg/min) of oxygen consumption.
The threshold value of mass, i.e. the first threshold value, is in a preferred embodiment always determined depending the height of a person. Preferably, the commonly used body weight index BMI (body mass index) is used, the index being calculated using the weight and height of a person with the formula $m/l^2$, in which m is the mass of person in kilograms and 1 is the height of a person in metres. This information is provided as pre-data that the user has typically entered into the apparatus executing the method. Thus, the first threshold value can be determined on the basis of a predetermined, usually fixedly set BMI value, when the height of a person is known. The threshold body weight index can be, e.g. 18.5, which corresponds to the lower limit of normal weight (WHO BMI classification). The precise value used in the analysis is selected on the basis of available reference data so that the results calculated on the basis of the analysis model are as close to the reference values as possible. Generally, the threshold value is selected in the body weight index range of 18 - 25.
According to a preferred embodiment determining the respiratory frequency comprises the following steps:

- determining the lengths of inter-beat intervals on the basis of heart rate data,
- calculating the difference between subsequent inter-beat intervals and classifying the difference as value A, if the difference is negative and as value B, if the difference is positive,
- calculating the Fourier transform for the produced time series,
- determining the respiratory frequency from the frequency response obtained with the Fourier transform.

It is further advantageous if the values A and B are selected so that no multiplication calculation is needed for the Fourier transform. A can be, for example, 0 and B can be 1. According to one application ventilation is calculated on the basis of respiratory frequency essentially with the formula:

$$\text{ventilation} = \text{respiratory frequency} * \text{vital capacity} * \text{correction factor,}$$

wherein the correction factor depends on the intensity of the exercise (again determined on the basis of heart rate or respiratory frequency or ventilation) and vital capacity is provided as pre-data typically depending on gender, age and height of the person.

Finally, energy consumption can be calculated with the formula:

$$\text{energy consumption} = b * \text{ventilation} * m_{eff}/m_{real,}$$

wherein b is a constant and $m_{eff}$ is the effective mass and $m_{real}$ the actual mass of the person. The constant b also contains the necessary unit conversion from a volume unit to an energy consumption unit.

An apparatus for determining the energy consumption during a person's physical exercise or after it comprises, according to one embodiment,

- means for measuring the heart rate or for importing a heart rate signal from an external heart rate sensor for providing heart rate data,
- a data processing unit for determining inter-beat intervals from the heart rate data and further for determining respiratory frequency and energy consumption by means of inter-beat intervals,
- a memory means for saving the pre-data related to the person and at least the first and second threshold value,

The data processing unit is arranged to

- select a first threshold value for the mass of a person, the value being saved to the memory means,
- select a second threshold value for the intensity of the exercise, the value being saved to the memory means,
- determine, whether the mass of the person is larger than the first threshold value,
- determine on the basis of the heart rate data whether the intensity of the exercise is lower than the second threshold value, and
- in case the intensity of the exercise is lower than the second threshold value and the mass of the person is larger than the first threshold value, to determine the energy consumption using a formula taking into account the deviation of the mass and the intensity of the exercise from the said first and second threshold value, correspondingly.

Considerable advantages are achieved by means of the invention. Especially, with it a more accurate energy consumption estimate can be produced at a certain mass and intensity level. The inventors have observed that current models typically overestimate the relative oxygen consumption at low intensities. This can be especially noticed when researching the energy consumption measurements of overweight persons, but the error exists to a degree for normal weight persons as well. The deficiencies of current models are probably due to the fact that reference measurements have almost always been made in short-duration sports situations. According to one explanation model overweight persons have a smaller amount of so-called "active mass" taking directly part in the energy consumptions via metabolism in the aerobic range of the exercise in relation to measured mass than normal-weight persons. The invention corrects this error source of known definitions by means of using a threshold value for mass (or body weight index) and thus creating a better estimate of energy consumption. This is a valuable piece of information especially for those on a diet with the goal of wanting to lower their body weight index, but also to other persons doing physical exercise.

During a high intensity exercise the determination of energy consumption using traditional methods is more reliable than in resting state as in this case the effect of systematic errors in relation to the correct energy consumption is smaller. In resting state or on low intensities the basic energy consumption is small, whereby errors are relatively larger. The embodiment of the invention taking into account the intensity of the exercise in the correction solves this problem as well.

According to one variation the decision concerning the need for correction is primarily made on the basis of the intensity of the exercise, not mass. In this case another threshold value is selected for the intensity of the exercise and if the intensity of the exercise is lower than the second threshold value, energy consumption is calculated with a formula taking into account the deviation of the mass of the person and/or the intensity of the exercise from the predefined values, such as the first and /or second threshold value.

In the following the embodiments of the invention are discussed in more detail with reference to the appended drawings.

**Brief Description of the Drawings**

Figure 1 illustrates the method according to one embodiment of the invention as a flow chart.
Figure 2 illustrates an example of vital capacity according to age for females and males.
Figure 3a exemplifies the change of the length of the inter-beat intervals aa descriptor in an exemplary exercise.
Figure 3b illustrates the inter-beat interval data processed according to image 3a and Fourier-transformed as well as determining the respiratory frequency.

Detailed description of embodiments

Definitions

The term "intensity" (of an exercise) means the degree of exertion of the exercise. Intensity can be measured via heart rate, respiratory frequency, ventilation or a mathematical derivative or combination of these.

The term "weight index" or "BMI" primarily means the commonly accepted (e.g. World Health Organization, http://apps.who.int/bmi/index.jsp?introPage=intro_3.html) and used definition of $m/l^2$, wherein m is the mass of the person in kilograms and 1 the height of the person in metres, but it is not limited at this. As an expert will understand, the body shape, fatness, obesity of a person can be described also via other indexes, such as ones determined by e.g. height and weight, and they are suitable for use in the present invention as well for determining the threshold weight of mass separately for each person.

The abbreviation "HR" is used for referring to the absolute heart rate and the abbreviation "hrr" refers to the relation of the difference between heart rate and resting heart rate to heart rate reserve, i.e. $hrr = (HR-HR_{rest}) / (HR_{max} - HR_{rest})$ (typically the unit is "percent of heart rate reserve", i.e. %hrr = 100% *hrr), wherein HR is the current heart rate, $HR_{rest}$ is the resting heart rate and $HR_{max}$ is the maximum heart rate.

"Inter-beat interval" means the temporal distance between two subsequent heartbeats from each other. As patent literature and other literature have disclosed a number of methods for recognizing heart rates, these methods are not discussed here in closer detail.

General description of the method

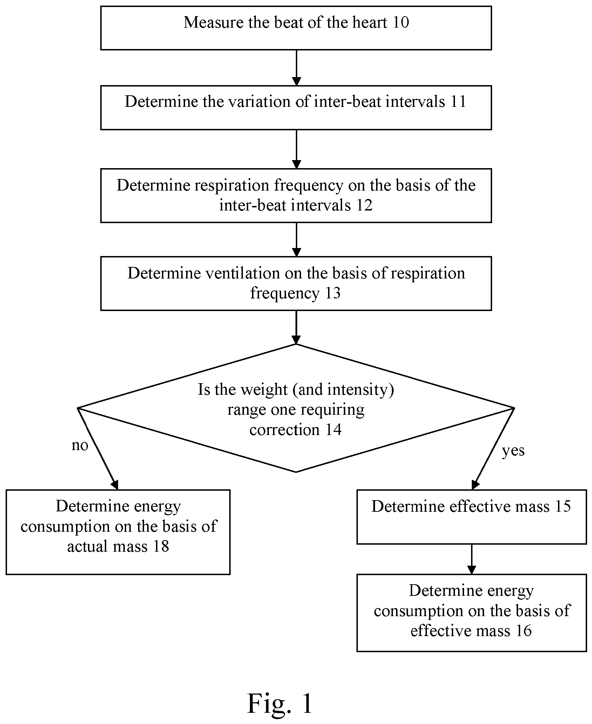

Figure 1 illustrates an example of carrying out the invention on a relatively general level. Heart rate is measured with a heart rate sensor, such as a heart rate belt arranged over the chest, in step 10. As one skilled in the art will understand, also other ways of recognizing heart rate, known in the field, can be used.

In step 11 the inter-beat intervals of subsequent heartbeats and further the inter-beat interval variation are determined from the heart rate data. The periodicity of the change of inter-beat intervals is indicative of respiratory frequency which is further determined in step 12.

In step 13, ventilation is determined based on the respiratory frequency and pre-data.

In step 14 it is determined on the basis of heart rate data and pre-data whether the weight and intensity range is one requiring effective mass correction. In case this is required, the process continues to step 15, in which the effective mass is calculated and further in step 16 energy consumption is calculated using the effective mass. In case the range is not one requiring effective mass correction, energy consumption is calculated directly on the basis of the actual mass of the person in step 18.

Calculation, i.e. steps 11 to 18 can be carried out in a suitable data processing unit, especially a computer, wrist computer or a mobile phone. Real-time energy consumption monitoring is preferably carried out in a wrist computer or a mobile phone. Most typically a computer is used for carrying out a post-analysis of the exercise.

Preferably the heart rate sensor is in wireless data transfer communication with the data processing unit.

The essential steps of the invention are discussed in more detail in the following.

Respiratory frequency

According to one embodiment respiratory frequency is essentially determined by means of method described in patent FI 121214 (US 7,803,117). According to this method the rate of a person's heart is monitored for providing a heart rate signal, respiratory frequency is determined on the basis of the periodicity of the temporal variation of the heart rate data contained by the heart rate signal so that the periodicity of the temporal variation of the heart rate data is determined in time level using time stamps created on the basis of the heart rate signal. Preferably respiratory frequency is determined so that a series comprising subsequent time points is formed of the time stamps, the periodicity of the series is determined, and the parameter describing respiration is determined on the basis of the sequence of the series. The sequence of the series can be determined by calculating the second derivative of the series and by looking for its zero points. For a more detailed description of the method reference is made to the above-mentioned patent publications.

According to an alternative embodiment respiratory frequency is determined as follows:

- the rate of a person's heart is measured by means of a suitable sensor,

- the lengths of inter-beat intervals are determined on the basis of heart rate data,

- the difference between subsequent inter-beat intervals is calculated and the difference is classified as value A, if the difference is negative and as value B, if the difference is positive, Typically A = 0 and B = 1. Thus the execution of Fourier transform in continued analysis can be optimized further.

- The Fourier transform of the time series assembled as described above is calculated. If the data consists of values 0 and 1, there is no need for windowing and multiplication.

- Values between which the largest value is selected are selected from the frequency response of the conversion of the previous step based on the heart rate data. Its location in the frequency space is selected to be respiratory frequency.

The largest advantage of such calculation for portable apparatuses is that multiplication is not needed and the calculation can easily and effectively be implemented with integer calculation. At the end of the disclosure there is a more detailed example about carrying out the calculation in practice. It is to be noted that the implementation described here is only suitable for cases in which it is desired to find out the periodicity of the data and it does not replace full Fourier transform. Additional advantages are that it is not necessary to separately correct heart rate data prior to analysis and it is not necessary to separately remove heart rate level changes therefrom. A change of heart rate level would mean an increase of mean heart rate as a result of e.g. increase of running speed. Such changes are seen in the frequency conversion of inter-beat intervals if they are not separately removed.

According to one embodiment, however, the following heart rate data correction is carried out:

- the difference diff between subsequent values is calculated, and

- if the difference is too large or too small (abs(diff) > quality trigger), 0 is selected as classification result.

Additionally, if ventilation data is not needed as such anywhere, calculation can be used only when it is observed that the intensity of the exercise in within the intensity range of the present invention, i.e. low enough.

It should be noted that the new respiratory frequency calculation method presented here is averaging in nature, i.e. the result is in this regard more reliable than the determination of periodicity directly in time level disclosed in patent FI 121214 (US 7,803,117).

Ventilation

At its simplest, ventilation is a product of respiratory frequency and depth of respiration (tidal volume). Estimating the respiratory depth requires data about vital capacity. Vital capacity can be estimated on the basis of literature. For example, the publication of American Thoracic Society, "Lung Function Testing: Selection of Reference Values and Interpretative Strategies", Am Rev Respir Dis 1991, American Thoracic Society, March 1991, can be used as a source. In this reference vital capacity has been tabulated as a function of gender, age and height.

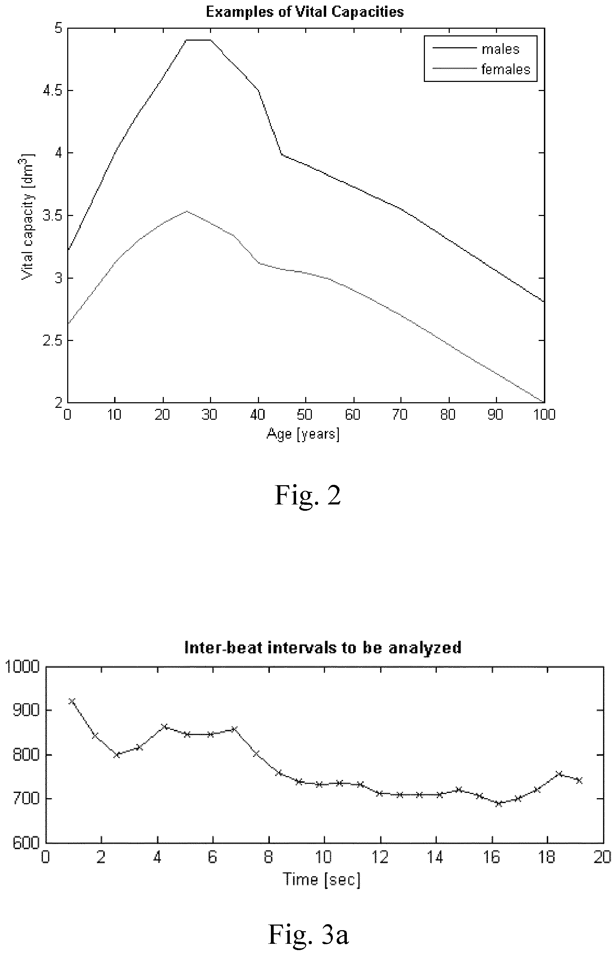

The above-mentioned literature reference contains general values that are most accurate in older age groups. More accurate estimates are available for especially younger age groups and they can be tabulated using reference material as well. Figure 2 shows a more accurate example of vital capacity as a function of age for males and females produced partly based on the above-mentioned reference and partly based on reference material. The vital capacity according to the invention can, if necessary, be further compensated for height.

When the vital capacity multiplied by respiratory frequency has been compared with the ventilation values of reference measurements, there was observed a need for a heart rate-dependent correlation function, which can be static and is provided e.g. as an average of reference measurements. According to one embodiment the factor depends on the value %hrr determined above. In other words

$$\text{ventilation} = \text{respiratory frequency} * \text{vital capacity} * \text{correction factor}(\%hrr)$$

Thus, when taking the above-mentioned issues into account, ventilation VE in this context depends on many factors, the most important of which are gender, age, height, %hrr, respiratory frequency.

Energy consumption in resting state and low intensity

According to one embodiment the level of basic metabolism, i.e. the BMR value per kilogram, is supposed to be constant, whereby the fixed estimate for oxygen consumption is 1 MET = 1 ml/kg/min.

According to a preferred embodiment a more accurate BMR value and further an oxygen consumption relating to said BMR value are used. For this purpose there are numerous formulae available from literature. For example, historically the most important are the Harris-Benedict equations from 1919:

$$\text{BMR\_males} = 13.7516 * m + 5.0033 * h - 6.775 * a + 66.473$$

$$\text{BMR\_females} = 9.5634 * m + 1.8496 * h - 4.6756 * a + 655.0955$$

In the above, m is weight in kilograms, h is height in centimetres and a is age in years.

It has, however, been noted that near resting state the above-mentioned method gives too high an oxygen consumption estimate for overweight persons.

According to the invention, this problem can be solved by determining threshold mass ($m_0$ (so-called "zero mass") for overweight persons. The accurate body weight index, BMI, for providing this data, can be determined by means of e.g. reference measurements and the difference between the values produced by the method and the reference values. It is also possible to use a BMI value on the range of normal weight 18.5 to 25. In testing it has been found that a relatively good value is a BMI value of about 19.

More specifically, the idea of the correction based on BMI is to select a threshold value for both low intensity (second threshold value) as well as the threshold mass (first threshold value) and to interpolate the zero mass so as to be the correct mass, when the intensity of the exercise changes from zero to this threshold value. This can be done through effective mass $m_{eff}$. In mathematical terms the effective mass at low intensities is

$$m_{eff} = m_0 + a*(m-m_0)*(I - I_0).$$

In the above, m is weight and intensity can be described by e.g. the above-mentioned unit %hrr, ventilation or other unit describing the intensity. $I_0$ is the selected threshold value for intensity. The factor a is a scaling constant.

When the intensity of the exercise is lower than the chosen threshold value for intensity ($I<I_0$), if BMI is larger than the threshold value (due to which $m>m_0$), effective mass $m_{eff}$ is used as basis for calculation, as is described below in more detail. On the other hand, if BMI is lower than the selected threshold value, ($m<m_0$), actual mass is used directly as mass.

Finally, the units $m_{eff}$ and ventilation described above are used for calculating an estimate for momentary energy consumption at low intensities.

$$E = vo2(\text{ventilation}) * m\_eff / 200.$$

The function vo2 about ventilation can be, for example

$$vo2 \ (ml/kg/min) = 0.385 \ (ml/l) * \text{ventilation} \ (l/min) / m_{real}.$$

This function will change accordingly (shape, factors) to fit the data better, as the reference database gets more accurate.

If intensity is higher than intensity _0, effective mass correction is preferably not made as described above, but instead the method described in e.g. patent FI 121214 (US 7,803,117) is used.

If no inter-beat interval data are available, BMR and BMI correction are applied directly to the calculated vo2 value (i.e. not ventilation corrected) at low intensities. The difference in these is that in resting state the heart rate reacts to other than performed work and can thus be seen as energy consumption in the basic method. This can be compensated by selecting in the basic method the effective mass so that in relation to the reference measurements the results are unbiased (i.e. averages are the same but regression is not as good as in a method improved with ventilation).

**Example**

This example illustrates, with computer code shown in tables 1 to 5, a practical execution of the invention in a simple manner having a small power consumption.

### Table 1. Initializing exemplary inter-beat interval data (values in milliseconds)

```
function sample_fDft
%
%
%
dataHere = [920 843 799 816 861 845 845 856 801 759 738 731 735 733 713 ...
    709 708 710 719 705 689 699 719 755 740 758];
fPwd = fDft(dataHere);
```

*Table 2. Initialization of variables and classification of inter-beat intervals*

```
function fPwd = fDft(d)
%
%
% Here the resolution in time domain is 50 ms. With N = 400 this means that
% there is 20 s of data in buffer. Below is the formula of the discrete
% Fourier transformation.
%
%            N
% X(k) = sum  x(n)*exp(-j*2*pi*(k-1)*(n-1)/N), 1 <= k <= N.
%           n=1
% This formula is used in the implementation below.


global sin_n cos_n

% Initialize variables. Cos_n and Sin_n are constants in real
% implementation.
F_s = 1/0.050; % 1/(50 ms)
N = 400;
freq=(0:N-1)*(F_s/N);
n = 0:(N-1);
cos_n = cos(2*pi*n/N);
sin_n = -sin(2*pi*n/N);
data = zeros(400,1);


% Take the first 20 s of incoming data in this example and classify the
% differences of the consecutive values. If the newest value is greater
% than the previous, fill the buffer with value A (here A = 1). Otherwise
% the buffered value is B (here B = 0).
d_prev = d(1);
index_prev = 0;
s = 0;
for i=1:max(size(d)),
    s = s + d(i);
    if s < 20000,
        index = mod( floor(s/50), 400 );
        if d(i) > d_prev,
            for k=index_prev+1:index,
                data(k) = 1;
            end
        end
        index_prev = index;
    else
        break;
    end
    d_prev = d(i);
end
```

*Table 3. Calculating the Fourier transform and determining and outputting the respiration frequency*

```
% The guidance to watch the correct frequency range can come from outside
% or it can be calculated based on the current incoming data. Here constant
% limits of 0 and 30 bpm are used.
ii = find(freq*60>0 & freq*60<30);
lowerFreqIndex = ii(1) - 1;
upperFreqIndex = ii(end) - 1;
fPwd = getPwd(lowerFreqIndex,upperFreqIndex,data);


% Calculate the respiration rate. Resolution can be enhanced by calculating
% the center of the mass of the power density peak. Here the location of
% the highest value is considered to be the respiration rate.
[m,iMax] = max(fPwd);
fprintf('Respiration rate is %d breaths per minute.\n',60*(iMax-1)*F_s/400);
```

## *Table 4. Plotting the curves*

```
% Plot the data and the power density function of the diference of that
% data
subplot(2,1,1);plot(cumsum(d(1:i-1))/1000,d(1:i-1),'x-');
title('\bf{Inter-beat intervals to be analyzed}');
xlabel('Time [sec]');

subplot(2,1,2);plot(freq(ii)*60,fPwd(ii));
title('\bf{Power density of the difference signal}');xlabel('Respiration rate [1/m
```

## *Table 5. Simplified Fourier transform function*

```
function fPwd = getPwd(lowerFreqIndex,upperFreqIndex,d)
%
% This implementation is valid only for values A=1 and B=0 (See the general
% explanation). Typically the calculation load here in this example is
% about (upperFreqIndex - lowerFreqIndex) * (N/2) i.e. about 2000
% summations (half of the values are zeroes). This is about the same as
% using FFT with the same data. The complexity of the FFT is O(N)=N*log(N),
% here this is about 2400. In FFT, one has to use, in general,
% multiplications, too. Furthermore, no windowing is used here. Also,
% fixed point arithmetic can be used easily in this kind of an
% implementation.
%
global sin_n cos_n
f=zeros(200,2);
for i=0:(max(size(d))-1),
    for j=lowerFreqIndex:upperFreqIndex,
        if d(i+1) ~= 0,
            indexHere = mod( i*j, 400 );
            f(j+1,1) = f(j+1,1) + cos_n(indexHere+1);
            f(j+1,2) = f(j+1,2) + sin_n(indexHere+1);
        end
    end
end
fPwd = f(:,1).^2+f(:,2).^2;
```

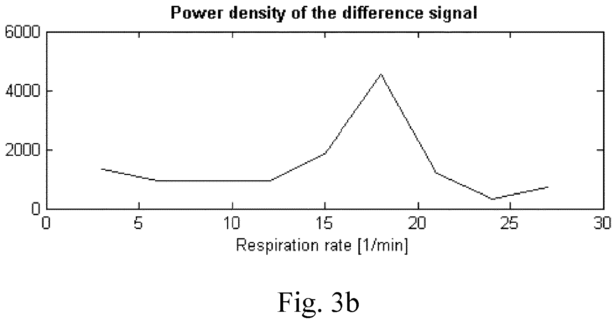

As can be seen from figure 3b, the peak value or center of mass of the curve and thus respiration frequency is at the point of 18 respirations per minute.

**Claims**

1. A method of estimating a person's energy consumption during or after a physical exercise on the basis of heart rate data, the method comprising

   - measuring heartbeat with a sensor,
   - determining the person's energy consumption on the basis of the heartbeat data,
   **characterized by**
   - selecting a first threshold value for the mass of a person,
   - selecting a second threshold value for an intensity of an exercise,
   - if the intensity of the exercise is lower than the second threshold value and the mass of the person is larger than the first threshold value, calculating the energy consumption using a formula taking into account the deviation of the mass and the intensity of the exercise from the said first and second threshold value, correspondingly,
   wherein said formula comprises a calculated variable that is an effective mass of the person, which is smaller than the actual mass, and wherein
   the effective mass approaches towards the value of the actual mass as the intensity of the exercise approaches towards the value of the second threshold value, and wherein
   energy consumption is calculated with said formula:

$$\text{energy consumption} = b * \text{ventilation} * m_{eff}/m_{real},$$

wherein b is a constant and $m_{real}$ is the actual and $m_{eff}$ the effective mass of the person, and ventilation is calculated using respiratory frequency, and
the effective mass $m_{eff}$ is defined essentially with the formula

$$m_{eff} = m_0 + a*(m - m_0)*(I - I_0),$$

wherein $I_0$ is the second threshold value, $m_0$ is the first threshold value, m is the mass of a person, I is the current intensity of the exercise and a is a constant.

2. A method according to claim 1, **characterized in that** the intensity and the second threshold value are determined based on heart rate frequency, respiratory frequency or ventilation.

3. A method according to claim 1 or 2, **characterized in that**

   - the person's respiratory frequency is determined using heart rate data,
   - energy consumption is determined using respiratory frequency.

4. A method according to claim 3, **characterized in that** determining the respiratory frequency comprises

   - determining the lengths of inter-beat intervals based on heart rate data,
   - calculating the difference between subsequent inter-beat intervals and classifying the difference as value A, if the difference is negative and as value B, if the difference is positive,
   - calculating the Fourier transform for the produced time series,
   - determining the respiratory frequency from the frequency response provided using the Fourier transform.

5. A method according to any of the previous claims, **characterized in that** ventilation is calculated on the basis of respiration frequency with the following formula:

$$\text{ventilation} = \text{respiratory frequency} * \text{vital capacity} * \text{correction factor,}$$

wherein the said correction factor depends on the intensity of the exercise and the vital capacity is provided as pre-data, typically depending on the gender, age and height of the person.

6. A method according to any of the previous claims, **characterized in that** the first threshold value is determined using a body mass index depending on the weight and height of the person.

7. A method according to any of the previous claims, **characterized in that** the first threshold value is selected so that it corresponds with a body mass index of 18 to 25 of the person.

8. An apparatus for determining energy consumption during or after a physical exercise of a person, the apparatus comprising

   - means for measuring heartbeat or for importing a heartbeat signal from an external heartbeat sensor for providing heartbeat data,
   - data processing unit for determining the length of inter-beat intervals from the heartbeat data and for further determining the energy consumption,
   - a memory means for saving pre-data relating to the person, such as mass or body mass index and at least a first threshold value describing the mass or body mass index of a person,
   **characterized in that**
   - the data processing unit is arranged to determine on the basis of the pre-data and the first threshold value whether the mass or body mass index of the person corresponds to a larger mass or body mass index than the first threshold value,
   - the memory means is arranged to also save a second threshold value describing the intensity of the exercise,
   - the data processing unit is arranged to determine on the basis of heart rate data whether the intensity of the exercise is lower than the first threshold value, and
   - in case the intensity of the exercise is lower than the second threshold value and the mass of the person is larger than the first threshold value, the data processing unit is arranged to determine the energy consumption using a formula taking into account the deviation of the mass and the intensity of the exercise from the said first and second threshold value, correspondingly,
- wherein said formula comprises a calculated variable that is an effective mass of a person, which is smaller than the actual mass, and wherein the effective mass approaches towards the value of the actual mass as the intensity of the exercise approaches towards the value of the second threshold value,
and wherein energy consumption is calculated with said formula:

$$\text{energy consumption} = b * \text{ventilation} * m_{\text{eff}}/m_{\text{real}},$$

wherein b is a constant and $m_{\text{real}}$ is the actual and $m_{\text{eff}}$ the effective mass of the person, and ventilation is calculated using respiratory frequency, and
the effective mass $m_{\text{eff}}$ is defined essentially with the formula

$$m_{\text{eff}} = m_0 + a*(m-m_0)*(I - I_0),$$

wherein $I_0$ is the second threshold value, $m_0$ is the first threshold value, m is the mass of a person, I is the current intensity of the exercise and a is a constant.

9. An apparatus according to claim 8, **characterized in that** it is arranged to execute the method according to any of claims 1 to 7.

**Patentansprüche**

1. Verfahren zum Schätzen eines Energieverbrauchs einer Person während oder nach einer physischen Betätigung auf der Basis von Herzratendaten, wobei das Verfahren umfasst

- Messen von Herzschlag mit einem Sensor,
- Ermitteln des Energieverbrauchs der Person auf der Basis der Herzschlagdaten,
**gekennzeichnet durch**
- Auswählen eines ersten Schwellenwerts für die Masse einer Person,
- Auswählen eines zweiten Schwellenwerts für eine Intensität einer Betätigung,
- falls die Intensität der Betätigung niedriger als der zweite Schwellenwert ist und die Masse der Person größer als der erste Schwellenwert ist, Berechnen des Energieverbrauchs unter Verwendung einer Formel, die die Abweichung der Masse und die Intensität der Betätigung von dem ersten beziehungsweise zweiten Schwellenwert berücksichtigt,
wobei die Formel eine berechnete Variable umfasst, die eine effektive Masse der Person ist, die kleiner als die berechnete Masse ist, und wobei
die effektive Masse sich an den Wert der tatsächlichen Masse annähert, wenn die Intensität der Betätigung sich dem Wert des zweiten Schwellenwerts annähert und wobei
Energieverbrauch mit der Formel berechnet wird:

$$\text{Energieverbrauch} = b*\text{Beatmung}*m_{\text{eff}}/m_{\text{real}},$$

wobei b eine Konstante ist und $m_{\text{real}}$ die tatsächliche und $m_{\text{eff}}$ die effektive Masse der Person ist und Beatmung unter Verwendung der Atemfrequenz berechnet ist, und
die effektive Masse $m_{\text{eff}}$ im Wesentlichen mit der Formel definiert ist

$$m_{\text{eff}} = m_0+a*(m-m_0)*(I-I_0),$$

wobei $I_0$ der zweite Schwellenwert ist, $m_0$ der erste Schwellenwert ist, m die Masse einer Person ist, I die aktuelle Intensität der Betätigung ist und a eine Konstante ist.

**2.** Verfahren nach Anspruch 1, **dadurch gekennzeichnet, dass** die Intensität und der zweite Schwellenwert basierend auf Herzratenfrequenz, Atemfrequenz oder Beatmung ermittelt werden.

**3.** Verfahren nach Anspruch 1 oder 2, **dadurch gekennzeichnet, dass**

- die Atemfrequenz der Person unter Verwendung von Herzratendaten ermittelt wird,
- Energieverbrauch unter Verwendung von Atemfrequenz ermittelt wird.

**4.** Verfahren nach Anspruch 3, **dadurch gekennzeichnet, dass** Ermitteln der Atemfrequenz umfasst

- Ermitteln der Längen von Zwischenschlagintervallen, basierend auf Herzratendaten,
- Berechnen der Differenz zwischen nachfolgenden Zwischenschlagintervallen und Klassifizieren der Differenz als Wert A, falls die Differenz negativ ist, und als Wert B, falls die Differenz positiv ist,
- Berechnen der Fourier-Transformation für die erstellte Zeitabfolge,
- Ermitteln der Atemfrequenz aus dem Frequenzgang, der unter Verwendung der Fourier-Transformation bereitgestellt wird.

**5.** Verfahren nach einem der vorstehenden Ansprüche, **dadurch gekennzeichnet, dass** Beatmung auf der Basis von Atemfrequenz mit der folgenden Formel berechnet wird:

$$\text{Beatmung} = \text{Atemfrequenz} * \text{Vitalkapazität} * \text{Korrekturfaktor},$$

wobei der Korrekturfaktor von der Intensität der Betätigung abhängt und die Vitalkapazität als Vorabdaten bereitgestellt ist, typischerweise abhängig von dem Geschlecht, Alter und der Größe der Person.

**6.** Verfahren nach einem der vorstehenden Ansprüche, **dadurch gekennzeichnet, dass** der erste Schwellenwert unter Verwendung eines Körpermasseindex ermittelt wird, abhängig von dem Gewicht und der Größe der Person.

**7.** Verfahren nach einem der vorstehenden Ansprüche, **dadurch gekennzeichnet, dass** der erste Schwellenwert so ausgewählt wird, dass er einem Körpermasseindex von 18 bis 25 der Person entspricht.

**8.** Einrichtung zum Ermitteln von Energieverbrauch während oder nach einer physischen Betätigung einer Person, wobei die Einrichtung umfasst

- Mittel zum Messen von Herzschlag oder zum Importieren eines Herzschlagsignals von einem externen Herzschlagsensor zum Bereitstellen von Herzschlagdaten,
- Datenverarbeitungseinheit zum Ermitteln der Länge von Zwischenschlagintervallen aus den Herzschlagdaten und zum weiteren Ermitteln des Energieverbrauchs,
- ein Speichermittel zum Speichern von Vorabdaten bezüglich der Person, wie Masse oder Körpermasseindex, und mindestens eines ersten Schwellenwerts, der die Masse oder den Körpermasseindex einer Person beschreibt,
**dadurch gekennzeichnet, dass**
- die Datenverarbeitungseinheit eingerichtet ist, auf der Basis der Vorabdaten und des ersten Schwellenwerts zu ermitteln, ob die Masse oder der Körpermasseindex der Person einer größeren Masse oder einem größeren Körpermasseindex entspricht als dem ersten Schwellenwert,
- das Speichermittel eingerichtet ist, auch einen zweiten Schwellenwert zu speichern, der die Intensität der Betätigung beschreibt,
- die Datenverarbeitungseinheit eingerichtet ist, auf der Basis von Herzratendaten zu ermitteln, ob die Intensität der Betätigung niedriger als der erste Schwellenwert ist, und
- im Fall, dass die Intensität der Betätigung niedriger als der zweite Schwellenwert ist und die Masse der Person größer als der erste Schwellenwert ist, die Datenverarbeitungseinheit eingerichtet ist, den Energieverbrauch unter Verwendung einer Formel zu ermitteln, die die Abweichung der Masse und die Intensität der Betätigung von dem ersten beziehungsweise zweiten Schwellenwert berücksichtigt,
- wobei die Formel eine berechnete Variable umfasst, die eine effektive Masse einer Person ist, die kleiner als die tatsächliche Masse ist und wobei die effektive Masse sich dem Wert der tatsächlichen Masse annähert, wenn sich die Intensität der Betätigung dem Wert des zweiten Schwellenwerts annähert, und wobei Energieverbrauch mit der Formel berechnet wird:

$$\text{Energieverbrauch} = b*\text{Beatmung}*m_{eff}/m_{real},$$

wobei b eine Konstante ist und $m_{real}$ die tatsächliche und $m_{eff}$ die effektive Masse der Person ist und Beatmung unter Verwendung von Atemfrequenz berechnet wird, und
die effektive Masse $m_{eff}$ im Wesentlichen mit der Formel definiert ist

$$m_{eff} = m_0 + a*(m-m_0)*(I-I_0),$$

wobei $I_0$ der zweite Schwellenwert ist, $m_0$ der erste Schwellenwert ist, m die Masse einer Person ist, I die aktuelle Intensität der Betätigung ist und a eine Konstante ist.

9. Einrichtung nach Anspruch 8, **dadurch gekennzeichnet, dass** sie eingerichtet ist, das Verfahren nach einem der Ansprüche 1 bis 7 auszuführen.

**Revendications**

1. Procédé d'estimation de la consommation d'énergie d'une personne pendant ou après un exercice physique sur la base de données de fréquence cardiaque, le procédé comprenant

   - la mesure du rythme cardiaque avec un capteur,
   - la détermination de la consommation d'énergie de la personne sur la base des données de rythme cardiaque,
   **caractérisé par**
   - la sélection d'une première valeur seuil pour la masse d'une personne,
   - la sélection d'une seconde valeur seuil pour une intensité d'un exercice,
   - si l'intensité de l'exercice est inférieure à la seconde valeur seuil et la masse de la personne est supérieure à la première valeur seuil, calculer la consommation d'énergie en utilisant une formule tenant compte de l'écart entre la masse et l'intensité de l'exercice à partir desdites première et seconde valeurs seuil, de manière correspondante,
   dans lequel ladite formule comprend une variable calculée qui est une masse effective de la personne, qui est inférieure à la masse réelle, et dans lequel
   la masse effective s'approche de la valeur de la masse réelle à mesure que l'intensité de l'exercice s'approche de la valeur de la seconde valeur seuil, et dans lequel
   la consommation d'énergie est calculée avec ladite formule :

$$\text{consommation d'énergie} = b*\text{ventilation}*m_{eff}/m_{réelle},$$

   dans lequel b est une constante et $m_{réelle}$ est la masse réelle et $m_{eff}$ la masse effective de la personne, et la ventilation est calculée en utilisant la fréquence respiratoire, et
   la masse effective $m_{eff}$ est définie essentiellement avec la formule

$$m_{eff} = m_0 + a*(m-m_0)*(I-I_0),$$

   dans lequel $I_0$ est la seconde valeur seuil, $m_0$ est la première valeur seuil, m est la masse d'une personne, I est l'intensité actuelle de l'exercice et a est une constante.

2. Procédé selon la revendication 1, **caractérisé en ce que** l'intensité et la seconde valeur seuil sont déterminées sur la base de la fréquence cardiaque, de la fréquence respiratoire ou de la ventilation.

3. Procédé selon la revendication 1 ou 2, **caractérisé en ce que**

   - la fréquence respiratoire de la personne est déterminée en utilisant des données de fréquence cardiaque,
   - la consommation d'énergie est déterminée en utilisant la fréquence respiratoire.

**4.** Procédé selon la revendication 3, **caractérisé en ce que** la détermination de la fréquence respiratoire comprend

- la détermination des longueurs d'intervalles entre les battements cardiaques sur la base des données de fréquence cardiaque,
- le calcul de la différence entre des intervalles entre les battements cardiaques suivants et la classification de la différence en tant que valeur A, si la différence est négative et en tant que valeur B, si la différence est positive,
- le calcul de la transformée de Fourier pour les séries chronologiques produites,
- la détermination de la fréquence respiratoire à partir de la réponse en fréquence fournie en utilisant la transformée de Fourier.

**5.** Procédé selon l'une quelconque des revendications précédentes, **caractérisé en ce que** la ventilation est calculée sur la base de la fréquence respiratoire avec la formule suivante :

$$\text{ventilation} = \text{fréquence respiratoire} * \text{capacité vitale} * \text{facteur de correction,}$$

dans lequel ledit facteur de correction dépend de l'intensité de l'exercice et la capacité vitale est fournie en tant que prédonnée, habituellement en fonction du sexe, de l'âge et de la taille de la personne.

**6.** Procédé selon l'une quelconque des revendications précédentes, **caractérisé en ce que** la première valeur seuil est déterminée en utilisant un indice de masse corporelle en fonction du poids et de la taille de la personne.

**7.** Procédé selon l'une quelconque des revendications précédentes, **caractérisé en ce que** la première valeur seuil est choisie de sorte qu'elle corresponde à un indice de masse corporelle allant de 18 à 25 de la personne.

**8.** Appareil de détermination de la consommation d'énergie pendant ou après un exercice physique d'une personne, l'appareil comprenant

- un moyen de mesure du rythme cardiaque ou d'importation d'un signal de rythme cardiaque à partir d'un capteur externe de rythme cardiaque pour fournir des données de rythme cardiaque,
- une unité de traitement des données pour déterminer la longueur d'intervalles entre les battements cardiaques à partir de données de rythme cardiaque et pour déterminer en outre la consommation d'énergie,
- un moyen de mémoire pour enregistrer des prédonnées relatives à la personne, telles que la masse ou l'indice de masse corporelle et au moins une première valeur seuil décrivant la masse ou l'indice de masse corporelle d'une personne,
**caractérisé en ce que**
- l'unité de traitement des données est agencée pour déterminer sur la base des pré-données et de la première valeur seuil si la masse ou l'indice de masse corporelle de la personne correspond à une masse ou à un indice de masse corporelle supérieur(e) à la première valeur seuil,
- le moyen de mémoire est agencé pour également enregistrer une seconde valeur seuil décrivant l'intensité de l'exercice,
- l'unité de traitement des données est agencée pour déterminer sur la base des données de fréquence cardiaque si l'intensité de l'exercice est ou non inférieure à la première valeur seuil, et
- dans le cas où l'intensité de l'exercice est inférieure à la seconde valeur seuil et la masse de la personne est supérieure à la première valeur seuil, l'unité de traitement des données est agencée pour déterminer la consommation d'énergie en utilisant une formule tenant compte de l'écart entre la masse et l'intensité de l'exercice à partir desdites première et seconde valeurs seuil, de manière correspondante,
- dans lequel ladite formule comprend une variable calculée qui est une masse effective d'une personne, qui est inférieure à la masse réelle, et dans lequel la masse effective s'approche de la valeur de la masse réelle à mesure que l'intensité de l'exercice s'approche de la valeur de la seconde valeur seuil,
et dans lequel la consommation d'énergie est calculée avec ladite formule :

$$\text{consommation d'énergie} = b * \text{ventilation} * m_{eff}/m_{réelle,}$$

dans lequel b est une constante et $m_{réelle}$ est la masse réelle et $m_{eff}$ la masse effective de la personne, et la ventilation est calculée en utilisant la fréquence respiratoire, et
la masse effective $m_{eff}$ est définie essentiellement avec la formule

$$m_{eff} = m_0 + a*(m-m_0)*(I-I_0),$$

dans lequel $I_0$ est la seconde valeur seuil, $m_0$ est la première valeur seuil, m est la masse d'une personne, I est l'intensité actuelle de l'exercice et a est une constante.

9. Appareil selon la revendication 8, **caractérisé en ce qu'**il est agencé pour réaliser le procédé selon l'une quelconque des revendications 1 à 7.

Measure the beat of the heart 10

↓

Determine the variation of inter-beat intervals 11

↓

Determine respiration frequency on the basis of the inter-beat intervals 12

↓

Determine ventilation on the basis of respiration frequency 13

↓

Is the weight (and intensity) range one requiring correction 14

no ↓

Determine energy consumption on the basis of actual mass 18

yes ↓

Determine effective mass 15

↓

Determine energy consumption on the basis of effective mass 16

# Fig. 1

Fig. 2

Fig. 3a

Fig. 3b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5810722 A
- US 20050209521 A
- EP 0072235 A2
- WO 2009131664 A2
- US 7431696 B1
- US 20090018405 A1
- US 20070265534 A1
- FI 121214
- US 7803117 B

### Non-patent literature cited in the description

- Poor agreement between continuous measurements of energy expenditure and routinely used prediction equations in intensive care unit patients. **REID et al.** CLINICAL NUTRITION. CHURCHILL LIVINGSTONE, 01 October 2007, vol. 26, 649-657
- Lung Function Testing: Selection of Reference Values and Interpretative Strategies. Am Rev Respir Dis 1991. American Thoracic Society, March 1991